# EUROPÄISCHE PATENTANMELDUNG

(11) **EP 4 101 973 A1**
(43) Veröffentlichungstag der Anmeldung: **14.12.2022**
(21) Anmeldenummer: 22168945.8
(22) Anmeldetag: 20.04.2022
(51) Int. Cl.: D06M 11/83, A61L 15/46, A61L 15/18, A61L 15/44, A41D 13/00, A41D 31/30, A47C 31/00, D06M 16/00, D06M 101/06

(54) **WASCHPERMANENTE BIOAKTIVE CELLULOSEFASER MIT ANTIBAKTERIELLEN UND ANTIVIRALEN EIGENSCHAFTEN**

(30) Priorität: 21.04.2021 DE 102021110053
(71) Anmelder: Smartpolymer GmbH, 07407 Rudolstadt (DE)
(72) Erfinder: Wendler, Frank, 07407 Rudolstadt (DE); Schulze, Thomas, 07407 Rudolstadt (DE); Bauer, Janine, 07407 Rudolstadt (DE)
(74) Vertreter: Plate, Jürgen

(57) **Zusammenfassung**

Offenbart ist eine cellulosische Faser, die mit einer biologisch aktiven Substanz beladen ist, sowie ein Verfahren zu deren Herstellung. Das Verfahren beiinhaltet die folgenden Schritte: a) Herstellen einer mit Ionentauscher beladenen cellulosischen Faser, b) Nachbehandeln der so hergestellten Faser mit einer wässrigen Lösung eines Metallsalzes, das antibakterielle Wirksamkeit und/oder antivirale Wirksamkeit aufweist, und c) Nachbehandeln der beladenen Faser mit einer wässrigen Fixierlösung, wodurch das Metallsalz in eine wasserunlösliche Form überführt wird. Die so hergestellten cellulosischen Fasern lassen sich mit textilen Fasern mischen und zu textilen Flächengebilden verarbeiten. Sie können auch zur Herstellung von Wundauflagen, Hygieneartikeln, Spezialpapieren, Verpackungs- oder Filtermaterialen verwendet werden.

## Beschreibung

### [Beschreibung]

Die Erfindung betrifft ein Verfahren zur Herstellung von cellulosischen Formkörpern mit lang anhaltenden, waschpermanenten antibakteriellen und antiviralen Eigenschaften. Daraus ergeben sich Anwendungen in der Medizin (Laborkittel), Hygiene (Mund- und Nasenmaske), Bekleidung (Sport- und Freizeitbereich)und Bettwaren (Matratzen).

### [Stand der Technik]

Es ist bekannt, dass Schwermetallionen, wie z. B. Bismut-, Silber-, Quecksilber, Kupfer-, Zink-, Zinn- oder Zirkoniumionen gegen pathogene Keime, wie Algen, Bakterien, Parasiten, Pilze, Prionen, Protisten, Viren oder Viroide wachstumshemmend bis abtötend wirken (R.B. Thurman u. C.P. Gerba: The molecular mechanismus of copper and silver ion disinfection of bacteria and viruses. CRC Critical Reviews in Environmental Control, Vol. 18, Issue 4 (1989), S. 295-315). Für eine bakterizide Wirkung sind Silber,- Kupfer- und Zinkionen von besonderem Interesse. Der entscheidende Vorteil dieser Ionen im Vergleich zu anderen bakterizid wirkenden Metallionen, wie z. B. Hg²⁺, ist die weitestgehende Unempfindlichkeit des menschlichen Metabolismus ihnen gegenüber. Die bakterizid wirkende Konzentration wird bei Silber mit 0,01 - 1 mg/l, Kupfer 0,1 - 1 mg/l angegeben (Ullman's Encyclopedia of Industrial Chemistry (5. Aufl.), VCH 1993, Volume A 24, S. 160).

Darüber hinaus wurde für Kupfer eine sehr effektive antivirale Wirkung nachgewiesen (S.L. Warnes, Z.R. Little u. C.W. Keevil: Human Coronavirus 229E Remains Infectious on Common Touch Surface Materials. mBIO 6(6):e01697-15 (2015)). Kupfer ist in der Lage, ein breites Spektrum an unbehüllten (z. B. Norovirus) und behüllten (z. B. Influenza, SARS CoV-2) Viren zu deaktivieren bzw. abzutöten. Sowohl Cu(II)- als auch Cu(I)-Ionen sind in der Lage, die Zellwand des Mikroorganismus über eine Peroxidation zu beschädigen. Dabei spielen Cu(I)-Ionen eine besondere Rolle, da sie mittels Fenton-Reaktion Hydroxylradikale generieren, welche Zellproteine zerstören können. Weiterhin führen Anbindungen beider Ionen an die DNA des Mikroorganismus zu genetischen Störungen, inklusive der Beschädigung der sogenannten Spike-Proteine an der Oberfläche des Virus.

Diese Wirkung der Schwermetalle, insbesondere Silber, Kupfer, Zink, wird seit langer Zeit in unterschiedlichsten Bereichen angewendet. So werden sensible Teile von medizinischen Geräten und Ausrüstungen oder Oberflächen mit hochfrequentem Kontakt, wie Geländer oder Türklinken, mit Kupfer oder Messing beschichtet. Für die Herstellung von Textilien wird vorwiegend das oberflächliche Auftragen (Imprägnieren, galvanische Ablagerung, Plasmabeschichtung, Gasphasenabscheidung) dieser Metalle genutzt. Eine weitere Möglichkeit ist das Einbringen von mit Metallionen dotierten Zeolithen oder Keramiken. Weiterhin ist die Herstellung von Garnen aus nicht metallhaltigen Fasern kombiniert mit Filamenten aus elementarem Silber oder Kupfer möglich.

EP2747792 verwendet synthetische Fasern, die vor der Extrusion mit Kupferionen gemischt werden. Die Ionen liegen in Form eines Kolloids in fester oder flüssiger Form vor.

In der DE69633817T2 wird eine Faser aus Acrylnitril mit Methylacrylat und ggf. mit Natriummethallylsulfonat hergestellt. Diese wird mit Hydrazin vernetzt und anschließend mit NaOH hydrolisiert zum Zwecke der Einführung von Carboxylgruppen. Nach der Aufbringung von Metallionen werden diese in einem fünften Schritt mittels Reduktionsmittel und Wärmebehandlung in die Faser präzipitiert. Diese Vorgehensweise ist sehr aufwändig und chemikalienintensiv. Bezüglich des Kupfers ist keine Reduktion zum Kupfer(I)-oxid vorgesehen, welches, wie oben beschrieben, für eine antivirale Wirkung prädestiniert ist.

EP2371893 beschreibt eine filmbildende Suspension aus nanoskaligen Cellulosefasern, einem polyvalenten Metall und einer flüchtigen Base.

In der DE69219821T2 werden Cellulosefasern erst mit einer Metallsalzlösung und anschließend mit einer Polycarbonsäurelösung behandelt. In einem vierten Arbeitsschritt erfolgt eine Hitzebehandlung bei 160°C zur Anbindung der Metallionen. Es wurde nach 10 Waschzyklen eine antibakterielle Wirkung gegen *Staphylococcus aureus* festgestellt. Sowohl eine antibakterielle Wirkung gegen *Klebsiella pneumoniae* als auch eine antivirale Wirkung nach 50 Waschzyklen wurde nicht beschrieben.

Aus CN107881763 ist die Einlagerung von nanoskaligem Kupferoxid zusammen mit Chitosan in eine Cellulosefaser bekannt. Die Synergie von Kupferoxid und Chitosan ergibt eine starke antibakterielle Wirkung. Die nicht näher angegebene hohe Waschpermanenz bezieht sich auf wiederholte Wäschen mit Ethanol und Wasser.

Ein weiterer Weg ist der Einbau einer natürlichen oder synthetischen Zweitkomponente in eine Cellulosefaser.

In DE10140772 werden Lyocell-Formkörper beschrieben, die Algen enthalten. Diese zeigen ein Sorptionsvermögen speziell für Schwermetallionen. Allerdings wird nur eine geringe Menge an Metallionen gebunden.

Die DE19917614 beschreibt die Herstellung von cellulosischen Formkörpern auf Polystyrol- oder Polyacrylatharzbasis mit hohem Absorptionsvermögen für Anionen und Kationen für die Verwendung als textile Ionentauschermaterialien. Jedoch werden keine Angaben zur Permanenz der Ionenbindung im textilen Gebrauch angegeben, da nur eine Einmal-Anwendung vorgesehen ist, z.B. als Zigarettenfilter und Haushaltsartikel.

Die oben erwähnte EP2747792 verwendet in einer Ausführungsform auch anteilig superabsorbierende Polymere (SAP) für Wundauflagen, die in Kombination mit Kupferionen Wundsekrete effektiv absorbieren und bakterizid wirken.

In der Literatur (M. Turalija, P. Merschak, B. Redl, U. Griesser, H. Duelli and T. Bechtold: Journal of Materials Chemistry / B, 2015, 3, 5886-5892) wird das Imprägnieren von textilen Polyester-Geweben mittels einer Suspension, bestehend aus Kupfer(I)oxid-Partikeln, einem Bindemittel und einem Dispergator, beschrieben, welches eine antibakterielle Wirksamkeit, auch nach zehn Wäschen, besitzt. Zehnmaliges Waschen des behandelten Gewebes erfolgte in 250 ml - Flaschen mittels Waschlösung über 30 min bei 60°C.

Der Erfindung liegt die Aufgabe zugrunde, ein Verfahren zur Herstellung von cellulosischen Formkörpern zu entwickeln, mit dem Schwerpunkt einer lang anhaltenden antibakteriellen und antiviralen Wirkung, für den Einsatz in der Medizin, Hygiene und Bekleidung. Eine weitere Aufgabe besteht darin, ein Depot des Wirkstoffes in der Faser aufzubauen, welches textilen Verarbeitungsschritten weitestgehend widersteht, sowie den Gebrauchsanforderungen eines Textils nachkommt. Letztere Aufgabe ist mit einer Waschpermanenz über 50 Wäschen verbunden. Des Weiteren sollen die nach dem erfindungsgemäßen Verfahren hergestellten Formkörper, insbesondere Fasern und Folien, so geschaffen werden, dass sie auf Grund ihres hohen Absorptionsvermögens zur Herstellung von Wundauflagen, Hygieneartikeln, Spezialpapieren, Verpackungs- sowie Filtermaterialien geeignet sind. Schließlich sollen Verbundstoffe aus Mischungen mit anderen Faserstoffen herstellbar werden. Dabei soll durch Verwendung der mit Wirkstoff beladenen Faser z. B. ein intrinsischer Faserschutz für Filter- und Geovliese, durch fungizide Wirkung, möglich sein. Ein weiteres Ziel der Erfindung ist es, das Verfahren zur Herstellung dieser cellulosischen Formkörper auf wenige Arbeitsschritte zu reduzieren.

Diese Aufgabe wird dadurch gelöst, dass cellulosische Formkörper mit hohem Absorptionsvermögen nach dem Trocken-Nassextrusionsverfahren, wie z. B. aus der DE19917614 bekannt (Ionentauscherfasern), mit ionischen Wirkstoffen beladen werden. Durch einen Nachbehandlungsschritt wird der Wirkstoff derart verändert, dass das in der Faser oder Folie aufgebaute Wirkstoffdepot in der Lage ist, diese Wirkstoffe entsprechend ihrer Gleichgewichtskonzentration über einen Zeitraum von mindestens 50 industriellen Standardwäschen so abzugeben, dass auch nach mindestens 50 Wäschen die antivirale und/oder antibakterielle Wirksamkeit gegeben ist. Die Gleichgewichtskonzentration ist über das Verhältnis der tatsächlichen Beladung zur Gesamtkapazität regelbar.

Kurze Beschreibung der Zeichnungen:
Abb. 1 zeigt ein durch Röntgenweitwinkelstreuung (WAXS) erzeugtes Spektrum einer mit Silberionen beladenen Faser, in der die Silberionen nachträglich mit Natriumchlorid fixiert wurden (oben) und zum Vergleich ein Spektrum von reinem Silberchlorid (unten).
Abb.2 zeigt den Anteil an Kupfer in einer mit Kupfersulfat beladenen SAP-Faser, mit und ohne Fixierung durch eine Natriumcarbonat-Lösung in Abhängigkeit von der Anzahl an durchgeführten Wäschen.
Abb. 3 zeigt das WAXS-Spektrum einer Faser, die mit zweiwertigen Kupferionen beladen und anschließend mit einer basischen Glucoselösung behandelt wurde (oben) und zum Vergleich ein Spekrum von reinem Kupfer(I)-oxid (unten).

Mit Waschpermanenz ist gemeint, dass die erfindungsgemäße Faser auch nach mindestens 50 industriellen Standardwäschen nach DIN EN ISO 6330 noch so viel aktive Substanz enthält, dass die biozide Wirkung weiterhin in vollen Umfang vorhanden ist. Die antivirale Wirksamkeit wird nach der Norm ISO 18184:2019-6 bestimmt. Die antibakterielle Wirksamkeit wird nach der Norm DIN EN ISO 20743:2013 bestimmt.

Mit Absorptionsvermögen ist die Aufnahme von Metallionen in der Faser gemeint. Es wird nach Norm DIN 54403:2009 bestimmt. Entsprechend der DE19917614 ist die Absorptionskapazität abhängig von der Art und Menge des inkorporierten Ionentauschers. Nach der bevorzugten Ausführungsform des erfindungsgemäßen Verfahrens enthält die zur Extrusion eingesetzte Spinnlösung 1 bis 200 Masse-%, vorzugsweise 10 bis 150 Masse-%, bezogen auf Cellulose, des Ionentauschers. Durch die Möglichkeit, hohe Konzentrationen an Ionentauscher in der Faser zu integrieren, können Wirkstoffdepots mit hohen Konzentrationen an Metallionen in der Faser angelegt werden. Wirkstoff im Sinne der Erfindung sind alle Metallsalze, welche wasserlöslich sind und sich somit in einem Nachbehandlungsschritt in die Faser einbringen lassen. Diese Einbringung erfolgt durch Wechselwirkung des Metallions mit den ionischen Gruppen des Ionentauschers in der Faser. Die Metallsalze müssen zusätzlich eine antivirale und/oder antibakterielle Wirksamkeit aufweisen. Zusätzlich müssen die Metallsalze in der Lage sein, sich durch einen weiteren Nachbehandlungsschritt in eine schwer wasserlösliche Form überführen zu lassen. Diese Überführung kann z.B. durch Zugabe der wässrigen Lösung eines Salzes erfolgen, dessen Anion mit dem Metallkation des Wirkstoffs eine in Wasser schwerlösliche Verbindung bildet. Es kann aber auch eine Nachbehandlung sein, durch die sich die Oxidationsstufe des Metallions ändert und sich dadurch ein schwer lösliches Metallsalz, Metalloxid oder elementares Metall bildet.

Metallsalze, welche als Wirkstoff in der Erfindung eingesetzt werden können, sind zum Beispiel wasserlösliche Silbersalze wie AgNO₃, AgF, wasserlösliche Zinksalze wie ZnSO₄, ZnI₂, ZnCl₂, ZnBr₂, Zn(ClO₃)₂ und wasserlösliche Kupfersalze wie CuSO₄, CuBr₂, Cu(ClO₃)₂, CuCl₂, CuSiF₆, Cu(NO₃)₂. Die Überführung in wasserunlösliche Formen geschieht durch Behandlung mit der wässrigen Lösung von Salzen, welche mit den Metallsalzen der Wirkstoffe schwerlösliche Salze bilden. Hierzu gehören wasserlösliche Salze der Halogene und Carbonate, aber auch Citrate, Phosphate, Salze von Fettsäuren und Sulfide. Geeignete Salze sind beispielsweise NaCl, NaF, NaBr, NaI, KF, KCl, KBr, KI, Na₂CO₃, NaHCO₃, Na₃PO₄, Na₂HPO₄, NaH₂PO₄, Na₂S, Natriumcitrat, Natriumstearat.

Alternativ können Wirkstoffe auch durch eine Redoxreaktion in eine andere Oxidationsstufe überführt werden, wodurch wasserunlösliche Verbindungen entstehen. Beispielsweise kann CuSO₄, welches in einem ersten Schritt an den mit Ionentauscher beladenen Fasern absorbiert wird, in alkalischem Milieu und in Gegenwart eines Reduktionsmittels zu Kupfer(I)-oxid reduziert werden, welches wasserunlöslich ist. Auch ist es möglich, Silber(I)-Salze durch Behandlung mit einer wässrigen Lösung aus Ammoniak und eines Reduktionsmittels zu elementarem Silber zu reduzieren, welches ebenfalls wasserunlöslich ist und in der Faser verbleibt.

Entsprechend DE10315749 liegen die Konzentrationen der Metalle, bevorzugt Silber, Kupfer und Zink, zweckmäßigerweise zwischen 0,005 g Metall / kg Faser bis >100 g Metall / kg Faser.

Als Ionentauscher werden Polymere auf Polystyrol- oder Polyacrylatbasis bevorzugt. Diese können sauer derivatisierte Styrol-Divinylbenzol- oder Acrylsäure-Divinylbenzol-Copolymerharze sein. Grundsätzlich können auch andere Trägermaterialien für die Austauschgruppen zum Einsatz kommen, wie z.B. Cellulose und Cellulosederivate.

Die mit Wirkstoff beladenen Fasern können mit unbeladenen Ionentauscherfasern gemischt werden, um die Wirkstoffkonzentration zu verringern bzw. zu steuern. Für die Herstellung von textilen Flächengebilden können die mit Wirkstoff beladenen Ionentauscherfasern mit anderen natürlichen und/oder synthetischen Fasern gemischt werden, wie z.B. Polyethylen-, Polypropylen-, Polyester-, Polyamid-, Polyacryl- oder cellulosischen Fasern.

Gegenstand der Erfindung ist demgemäß eine Stapelfaser, die vorzugsweise aus Cellulose besteht und nach einem Trocken-Nass-Prozess, wie dem Lyocell-Verfahren oder mit ionischen Flüssigkeiten als Lösungsmitteln, verformt werden kann. Denkbar ist auch das Viskoseverfahren als Herstellungsverfahren.

Das Beladen der Ionentauscherfaser erfolgt vorzugsweise nach einem Tauchverfahren, bei dem die Faser mit einer Salzlösung, die z.B. Silber-, Kupfer- oder Zinkionen enthält, getränkt wird. Die Faser wird anschließend mehrmals mit Wasser gewaschen und abgeschleudert. Nach Tränken in einem Avivierbad wird die Faser abermals abgeschleudert und getrocknet.

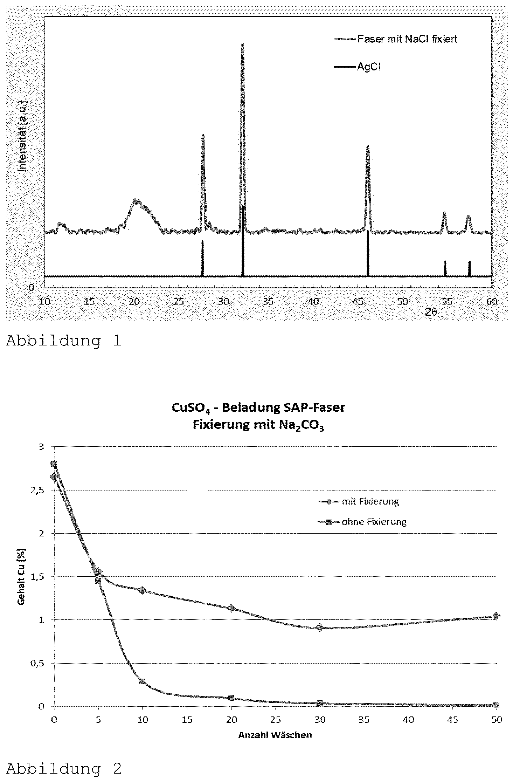

Waschversuche haben ergeben, dass diese Faser nur eine geringe Waschpermanenz aufweist. Schon nach 10 Wäschen ist ein Verlust von ca. 90% des Metalls zu verzeichnen. Daher wurde nach der bevorzugten Ausführungsform des erfindungsgemäßen Verfahrens die Vorgehensweise der Beladung um einen weiteren Arbeitsschritt erweitert: das Fixieren des Metallions in der Faser. Überraschenderweise hat sich gezeigt, dass durch die Behandlung der bereits beladenen Faser mit einer zweiten Salzlösung, enthaltend Chlorid- oder Carbonationen, eine in Wasser schwerlösliche Verbindung formiert wird, welche fester in der Faser gebunden ist und demzufolge der Faser eine deutlich höhere Waschpermanenz verleiht. Silberchlorid sowie Kupfer- und Zinkcarbonate sind in Wasser praktisch unlöslich. So konnten mittels Röntgenweitwinkelstreuung (WAXS) deutliche Hinweise auf das Vorhandensein von z.B. AgCl-Kristalliten in der Faser nachgewiesen werden. Abbildung 1 zeigt das WAXS-Spektrum einer mit Silberionen beladenen Faser mit zusätzlicher NaCl-Fixierung im Vergleich zum Spektrum der reinen AgCl-Verbindung.

Die deutlich höhere Waschpermanenz wurde mittels Haushaltswäsche bei 40°C mit üblichem Vollwaschmittel über 50 Waschzyklen nachgewiesen. Abbildung 2 zeigt als Beispiel die Messung der Kupfergehalte der Faser mit und ohne Na₂CO₃-Fixierung.

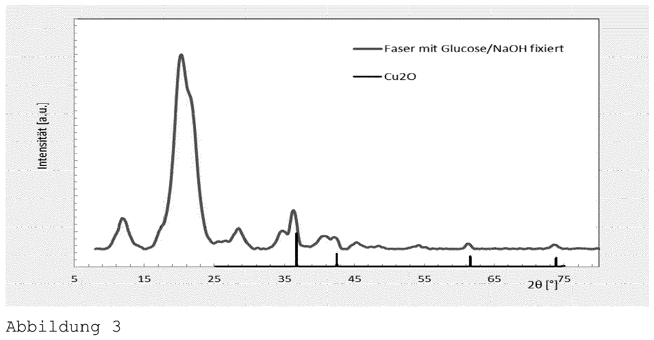

In einer weiteren Ausführungsform erfolgt die Fixierung mit gleichzeitiger Änderung der Oxidationszahl des Kupfers. Überraschenderweise konnte gefunden werden, dass eine mit zweiwertigen Kupferionen beladene Faser mit einer basischen Glucoselösung in eine Faser mit integriertem einwertigen Kupferoxid überführt werden kann. Auch dieses Kupfer(I)-oxid (Cu₂O) ist fest in der Faser eingebunden. Seine Kristallstruktur konnte durch WAXS-Messungen bestätigt werden (siehe Abbildung 3). Neben der höheren Waschpermanenz wird, wie oben beschrieben, eine effektive antivirale Wirkung erzielt. Da Kupfer(I)-Verbindungen an der Luft nicht stabil sind, werden sie sukzessive wieder zu zweiwertigem Kupfer oxidiert. Daher ist z.B. eine Beladung mit Cu₂O-Partikeln einer Faser nicht zielführend. Somit stellt die intrinsische Einführung des Cu₂O in die Cellulosefaser einen Schutz vor einer Oxidation dar. Eine langanhaltende (permanente) antivirale Wirkung ist durch dieses gebildete Cu₂O-Depot gewährleistet.

Trotz der festen Einlagerung des Metalls als Carbonat, Chlorid oder Oxid liegen noch genügend freie Ionen entsprechend oberflächlicher Gleichgewichtsreaktionen, z. B. Metallcarbonat ⇔ Metallion + Carbonation, im Cellulosegerüst vor. Durch die Wirkung der Cellulose als hydrophiles netzwerkbildendes Polymer mit einer Restfeuchte bis 15% ist ein Transport des Metallions vom Innern der Faser an die Oberfläche immer gewährleistet. Somit sind die Metallionen im Inneren und an der Oberfläche der Faser voll zugänglich, um ihre antibakterielle bzw. antivirale Wirkung auf Mikroorganismen zu entfalten. Auch über längere Gebrauchsphasen der Faser besteht immer ein Nachschub aus dem inneren Wirkstoffdepot an die Oberfläche. Die Gleichgewichtskonzentration der Metallionen ist ausreichend für den biologisch wirksamen Bereich. Es konnten sowohl die antibakterielle (Ag, Cu, Zn) als auch die antivirale Wirkung (Cu) nachgewiesen werden. Auch nach 50 Waschzyklen war nur ein geringer Verlust der antibakteriellen bzw. antiviralen Wirkung zu verzeichnen.

### Beispiele

Zur weiteren Verdeutlichung des erfindungsgemäßen Verfahrens dienen die folgenden Beispiele.

Prüfmethoden zur Bestimmung der Elementgehalte und zur Beurteilung der antibakteriellen und antiviralen Aktivität von cellulosischen Fasern:
Die antibakterielle Wirkung der Fasern wurde nach der Prüfvorschrift DIN EN ISO 20743 "Textilien - Bestimmung der antibakteriellen Wirksamkeit von textilen Produkten" ermittelt, indem eine definierte Anzahl an Bakterien in verdünnter Nährlösung auf die Fasern aufgebracht und diese für 24 h bei 37°C inkubiert wurden. Anschließend wurden die Bakterien mittels Ausschütteln abgelöst und die verbleibende Anzahl überlebender Bakterien mittels Ausplattierverfahren bestimmt. Aus den Logarithmen der erhaltenden Bakterienzellzahlen für eine nicht antibakteriell ausgerüstete Probe (Kontrollmaterial) sowie für die antibakteriellen Fasern wurde die Differenz berechnet, die ein Maß für die antibakterielle Wirksamkeit darstellt, wobei eine log 2-Reduktion eine gute antibakterielle Wirksamkeit bedeutet, eine log 3-Reduktion eine sehr gute Wirksamkeit.

Die antivirale Wirksamkeit wurde in Anlehnung an die Prüfvorschrift ISO 18184 "Textilien - Bestimmung der antiviralen Aktivität von Textilerzeugnissen" ermittelt. Dazu wurde der behüllte Bakteriophage phi6 als Surrogatvirus für die humanen, behüllten Viren Influenza A oder SARSA-CoV-2 verwendet und in einer definierten Anzahl auf die Fasern aufgebracht und diese für 2 h bei 25°C inkubiert. Anschließend wurden die Phagen mittels Ausschütteln abgelöst und die verbleibende Anzahl überlebender Phagen mittels Plaquetiterbestimmung ermittelt. Aus den Logarithmen der erhaltenden Plaquetiter für eine nicht antiviral ausgerüstete Probe (Kontrollmaterial) sowie für die antiviralen Fasern wurde die Differenz berechnet, die ein Maß für die antivirale Wirksamkeit darstellt, wobei eine log 2-Reduktion eine geringe antivirale Wirksamkeit bedeutet, eine log 3-Reduktion eine vollständige antivirale Wirksamkeit.

Die Bestimmung der Kupfer-, Silber- und Zinkgehalte erfolgte mittels ICP-OES entsprechend DIN EN ISO 11885 nach Mikrowellendruckaufschluss.

Die WAXS-Messungen wurden mit einem BRUKER-D8-Gerät der *Advance-Serie,* ausgerüstet mit einem positionsempfindlichen Zeilendetektor, in symmetrischer Transmission, durchgeführt. Zur Messung wurde Cu-K*_{α}*-Strahlung der Wellenlänge λ = 0,1542 nm (Dublett) bei einer Röhrenspannung von 40 kV, sowie 40 mA Anodenstrom und ausgefiltertem K_{β}-Anteil verwendet. Als Probenkörper wurden Tabletten einheitlicher Dichte und einer Dicke von 2 mm präpariert.

Verwendete Chemikalien:
Natriumpolyacrylat, quervernetzt (CAS: 9033-79-8, PRODUKT T 5066 F, Fa. Evonik)
Kupfer(II)sulfat-Pentahydrat (CAS: 7758-99-8, Reinheit ≥ 98%, z. B. Fa. VWR)
Natriumcarbonat (CAS: 497-19-8, Reinheit ≥ 98%, z. B. Fa. VWR)
Glucose-Monohydrat (CAS: 14431-43-7, Reinheit ≥ 98%, z. B. Fa. VWR)
Natriumchlorid (CAS-Nummer: 7647-14-5, Reinheit ≥ 98%, z. B. Fa. VWR)
Afilan^{®} RA (Octadecanamid, CAS-Nummer: 10220-90-3, Fa. Archroma)

### Beispiel 1

Die Ionentauscherfasern, hergestellt entsprechend DE19917614, mit einem Anteil von 15% Natriumpolyacrylat, werden mit einer Kupfersulfatlösung behandelt. Dazu werden 15 kg der Ionentauscherfaser mit entionisiertem Wasser gewaschen und anschließend mit einer 0,15 M wässrigen Kupfersulfatlösung beladen. Nach 20 min Verweilen in dieser Lösung unter intensivem Rühren werden die Fasern abgeschleudert und zentrifugiert. In einem zweiten Behandlungsbad werden die Fasern aviviert, unter Verwendung eines üblichen Weichmachers, z. B. Afilan^{®} RA. Die Fasern haben einen Titer von 6,7 dtex, eine Dehnung von 10% und eine Reißfestigkeit von 21 cN/tex. Die Kupferkonzentration beträgt 28.000 mg/kg Kupfer. Nach 50 Waschzyklen enthielten die Fasern noch 200 mg/kg Kupfer. Die Messung der antibakteriellen Wirkung gegenüber *Staphylococcus aureus* zeigte eine Reduktion von log 5,8, nach 50 Waschzyklen log 5,3; gegenüber *Klebsiella pneumoniae* eine Reduktion von log 5,8, nach 50 Waschzyklen log 5,5. Dies bedeutet gegenüber beiden Bakterientypen eine starke antibakterielle Wirksamkeit, die auch nach 50 Waschzyklen noch aufrechterhalten wird. Die Messung der antiviralen Wirkung gegen Pseudomonas sp. DSM 21482 ergab eine log 3,0-Reduktion, was einem starken antiviralen Effekt entspricht.

### Beispiel 2

Ionentauscherfasern hergestellt nach Beispiel 1, werden nach der Kupferbeladung zusätzlich in ein zweites Tauchbad mit einer 10 g/l-Natriumcarbonatlösung gegeben und darin für 20 Minuten gerührt. Anschließend wird abgeschleudert und zentrifugiert. In einem dritten Behandlungsbad werden die Fasern nach Beispiel 1 aviviert. Die Kupferkonzentration beträgt 26.500 mg/kg Kupfer. Nach 50 Waschzyklen enthielten die Fasern noch 10400 mg/kg Kupfer. Dies entspricht gegenüber der nicht fixierten Faser aus Beispiel 1 einer Erhöhung der Wiederfindungsrate nach 50 Wäschen von ca. 0,7 auf ca. 39%.

### Beispiel 3

Ionentauscherfasern hergestellt nach Beispiel 1, werden nach der Kupferbeladung zusätzlich in ein zweites Tauchbad mit einer Lösung enthaltend 10 g/l Glucose und 5 g/l NaOH gegeben und darin für 20 Minuten gerührt. Anschließend werden die Fasern mehrmals bis zur neutralen Reaktion gewaschen, abgeschleudert und zentrifugiert. In einem dritten Behandlungsbad werden die Fasern nach Beispiel 1 aviviert. Die Kupferkonzentration beträgt 7890 mg/kg Kupfer. Nach 50 Waschzyklen enthielten die Fasern noch 321 mg/kg Kupfer. Die antibakterielle Wirkung nach 50 Waschzyklen gegenüber *Staphylococcus aureus* zeigte eine Reduktion von log 4,7 und gegenüber *Klebsiella pneumoniae* eine Reduktion von log 4,4. Die antivirale Wirkung gegen *Pseudomonas sp.* DSM 21482 zeigte eine log 4,5 Reduktion, nach 50 Wäschen noch eine Reduktion von log 4,1. Dies entspricht einer starken antiviralen Wirksamkeit auch noch nach 50 Wäschen.

### Beispiel 4

Ionentauscherfasern hergestellt nach Beispiel 1, werden in einer anteiligen Mischung von 6% mit reinen Lyocell-Fasern zu einem Nadelvlies verarbeitet. Die antibakterielle Wirkung gegenüber *Staphylococcus aureus* konnte mit einer log 5,6-Reduktion bestimmt werden, nach 20 Waschzyklen betrug die Reduktion noch log 5,3. Gegenüber *Klebsiella pneumoniae* ergab sich eine log 5,9-Reduktion, nach 20 Waschzyklen eine log 4,4-Reduktion.

### Beispiel 5

Ionentauscherfasern hergestellt nach Beispiel 1, jedoch anstelle von Kupfersulfat mit 0,15 M wässriger Silbernitratlösung behandelt. Die Fasern haben einen Titer von 6,7 dtex, eine Dehnung von 11% und eine Reißfestigkeit von 23 cN/tex. Die Silberkonzentration beträgt 51.200 mg/kg Silber. Nach 50 Waschzyklen enthielten die Fasern noch 2150 mg/kg Silber.

### Beispiel 6

Ionentauscherfasern hergestellt nach Beispiel 5, werden nach der Silberbeladung zusätzlich in ein zweites Tauchbad mit einer 10 g/l-Natriumchloridlösung gegeben und darin für 20 Minuten gerührt. Anschließend wird entsprechend Beispiel 2 abgeschleudert, zentrifugiert und aviviert. Die Silberkonzentration beträgt 48.300 mg/kg Silber. Nach 50 Waschzyklen enthielten die Fasern noch 14.500 mg/kg Silber. Dies entspricht gegenüber der nicht fixierten Faser aus Beispiel 5 einer Erhöhung der Wiederfindungsrate nach 50 Wäschen von ca. 4 % auf ca. 30%. Die Messung der antibakteriellen Wirkung gegenüber *Staphylococcus aureus* zeigte eine Reduktion von log 6,0, nach 50 Waschzyklen log 5,8; gegenüber *Klebsiella pneumoniae* eine Reduktion von log 6,0, nach 50 Waschzyklen log 5,6. Dies bedeutet gegenüber beiden Bakterientypen eine starke antibakterielle Wirksamkeit, die auch nach 50 Waschzyklen noch aufrechterhalten wird.

## Patentansprüche

1. Verfahren zur Herstellung einer cellulosischen Faser, welche mit einer biologisch aktiven Substanz beladen ist, durch folgende Verfahrensschritte gekennzeichnet:
a. Herstellen einer mit Ionentauscher beladenen cellulosischen Faser;
b. Nachbehandlung der in a) hergestellten Faser mit einer wässrigen Lösung eines Metallsalzes, welches antibakterielle Wirksamkeit und/oder antivirale Wirksamkeit aufweist;
c. Nachbehandlung der in b) beladenen Faser mit einer wässrigen Fixierlösung, wodurch das Metallsalz in eine wasserunlösliche Form überführt wird.

2. Verfahren nach Anspruch 1, **dadurch gekennzeichnet, dass** es sich bei dem Metallsalz um wasserlösliche Silber-, Kupfer- oder Zinksalze, bevorzugt CuSO₄, AgNO₃ und ZnSO₄ handelt.

3. Verfahren nach Anspruch 1, **dadurch gekennzeichnet, dass** die wässrige Fixierlösung Salze mit Anionen ausgewählt aus F⁻, Cl⁻, Br⁻, I⁻, ClO₃⁻, ClO₄⁻, CO₃²⁻, HCO₃⁻, PO₄³⁻, HPO₄²⁻, H₂PO₄⁻, S²⁻, Citrat, oder Salzen von Fettsäuren enthält.

4. Verfahren nach Anspruch 1, **dadurch gekennzeichnet, dass** die wässrige Fixierlösung eine Base und ein Reduktionsmittel enthält.

5. Verfahren nach Anspruch 4, **dadurch gekennzeichnet, dass** die Base NaOH oder Ammoniak ist und das Reduktionsmittel ein Aldehyd.

6. Cellulosische Faser hergestellt nach einem Verfahren der Ansprüche 1-5.

7. Cellulosische Faser nach Anspruch 6, **dadurch gekennzeichnet, dass** sie nach Mischen mit textilen Fasern, wie Polyethylen-, Polypropylen-, Polyester-, Polyamid-, Polyacryl- oder cellulosischen Fasern zu textilen Flächengebilden verarbeitet wird.

8. Cellulosische Faser nach Anspruch 6, **dadurch gekennzeichnet, dass** sie zur Herstellung von Wundauflagen, Hygieneartikeln, Spezialpapieren, Verpackungs- sowie Filtermaterialien verwendet wird.

9. Cellulosische Faser nach Anspruch 6, **dadurch gekennzeichnet, dass** sie als intrinsischer Faserschutz für Filter- und Geovliese verwendet wird.
